# FASCICULE DE BREVET EUROPEEN

(11) **EP 1 066 817 B1**
(45) Date de publication et mention de la délivrance du brevet: **18.02.2004**
(21) Numéro de dépôt: 00401973.3
(22) Date de dépôt: 07.07.2000
(51) Int. Cl.: A61K 7/02, A61K 7/48

(54) **Composition de maquillage comprenant des fibres**
Fasern enthaltende Schminkzusammensetzung
Make-up composition containing fibers

(30) Priorité: 08.07.1999 FR 9908960
(43) Date de publication de la demande: 10.01.2001
(73) Titulaire: L'OREAL, 75008 Paris (FR)
(72) Inventeur: Bara, Isabelle, 75013 Paris (FR)
(74) Mandataire: Kromer, Christophe

(56) Documents cités:
- EP-A- 0 100 194
- EP-A- 0 336 900
- WO-A-00/06114
- WO-A-97/29734
- DATABASE CHEMICAL ABSTRACTS [en ligne] STN; abrégé 123: 237 559, XP002133921 & JP 07 196440 A (SHISEIDO CO., LTD) 1 août 1995 (1995-08-01)

## Description

La présente invention a pour objet un procédé de maquillage ou de soin de la peau comprenant l'application d'une composition cosmétique comprenant des fibres et une dispersion aqueuse de polymère filmogène. L'invention a aussi pour objet une composition cosmétique comprenant des fibres et une dispersion aqueuse de polymère filmogène de polyuréthane. Le procédé de maquillage et la composition sont plus particulièrement destinés au maquillage du corps, de la peau du visage, y compris les lèvres, des paupières, des oreilles (et notamment le lobe) d'êtres humains.

Les produits de maquillage sont couramment employés pour apporter de la couleur, mettre en valeur certaines parties de la peau ou des phanères, ou bien encore procurer un aspect brillant, mat, satiné à la peau ou aux phanères. Ces produits sont habituellement appliqués sous la forme d'une mince couche uniforme. Toutefois, lorsque la peau présente des imperfections comme des taches, des boutons ou des cicatrices, ces imperfections ne sont pas bien camouflées du fait de la minceur du maquillage, notamment parce que le relief de ces imperfections ressort malgré le maquillage et n'est pas uniformisé.

Par ailleurs, avec l'évolution de la mode, les consommateurs, plus exigeants, recherchent de nouveaux produits de maquillage permettant d'obtenir des effets de maquillage originaux ou particuliers, et notamment conférant des transformations visibles du visage ou du corps maquillés.

Il est connu du document JP-A-7-196440 d'utiliser des fibres dans une composition de maquillage de la peau pour conférer à la peau un toucher velouté. Toutefois, les fibres dans ce type de composition n'adhèrent pas bien sur la peau et ont tendance à s'éliminer dans le temps. Une telle composition ne convient donc pas pour camoufler efficacement et durablement les imperfections de la peau.

La présente invention a donc pour but de proposer une composition de maquillage permettant d'obtenir un maquillage bien adhérent sur la peau et camouflant.

Les inventeurs ont découverts qu'un tel maquillage pouvait être obtenu en utilisant des fibres associées à un polymère filmogène en dispersion aqueuse. Le maquillage obtenu présente une bonne tenue, notamment il est résistant aux frottements et à l'eau. Les fibres ont une bonne cohésion dans le film de polymère et permet ainsi de camoufler les imperfections de la peau maquillée, durablement.

Le document WO-A-97 29734 concerne l'utilisation d'un polymère filmogène en solution aqueuse ou en dispersion aqueuse et d'une suspension aqueuse de microfibrilles d'origine naturelle comme agent de revêtement composite des cheveux, des cils, des sourcils, ou des ongles dans et pour la préparation d'une composition cosmétique ou dermatologique.

Il est connu des documents JP-A-57-158714, JP-A-9-263518 et JP-A-7-179323 des compositions de mascara comprenant des fibres et une dispersion aqueuse de polymère acrylique ou d'acétate de vinyle. Toutefois, ces documents ne suggèrent nullement d'utiliser ces compositions pour la peau pour en camoufler les imperfections. En particulier, les exigences quant au film déposé sont différentes : un film formé sur la peau ou les lèvres doit pouvoir suivre leurs mouvements.

De façon plus précise, la présente invention a pour objet un procédé cosmétique de maquillage ou de soin de la peau, comprenant l'application sur la peau d'une composition cosmétique contenant, dans un milieu physiologiquement acceptable, des fibres, caractérisé par le fait que la composition comprend un polymère filmogène sous forme de particules en dispersion aqueuse.

L'invention a également pour objet une composition cosmétique comprenant, dans un milieu physiologiquement acceptable, un polymère filmogène sous forme de particules en dispersion aqueuse et des fibres, caractérisée par le fait que le polymère filmogène est un polyuréthane.

L'invention a aussi pour objet l'utilisation de fibres et de polymère filmogène sous forme de particules en dispersion aqueuse dans une composition cosmétique de maquillage de la peau pour obtenir un maquillage de bonne tenue et/ou résistant à l'eau et/ou aux frottements et/ou camouflant la peau.

En plus des avantages mentionnés ci-dessus, la composition selon l'invention confère un maquillage présentant un effet de tissu, comme le velours, la dentelle ou la fourrure. Ce maquillage permet notamment d'imiter un vêtement.

Les fibres utilisables pour le procédé de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 50 mm, de préférence de 10 nm à 5 mm et mieux de 0,1 mm à 1,5 mm. Leur section peut être comprise dans un cercle de diamètre allant de 2 nm à 1 mm, de préférence allant de 20 nm à 500 µm et mieux de 500 nm à 20 µm. Le poids des fibres est souvent donné en denier ou décitex.

Les fibres peuvent être celles utilisées dans la fabrication des textiles et notamment des fibres de soie, de coton, de laine, de lin, des fibres de cellulose (ou rayonne) notamment extraites du bois, des légumes ou des algues, de polyamide (Nylon ®), de viscose, d'acétate notamment d'acétate de rayonne, de poly-(p-phénylène-téréphtalamide) (ou aramide) comme le Kevlar ®, de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon ®), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

Par ailleurs, les fibres peuvent être traités ou non en surface, enrobées ou non. Comme fibres enrobées utilisables dans l'invention, on peut citer des fibres de polyamides enrobées de sulfure de cuivre pour un effet anti-statique (par exemple le R-STAT de chez Rhodia) ou un autre polymère permettant une organisation particulière des fibres (traitement de surface spécifique) ou traitement de surface induisant des effets de couleurs/hologrammes (fibre Lurex de chez Sildorex, par exemple).

De préférence, on utilise des fibres d'origine synthétiques et en particulier des fibres organiques, comme celles utilisées en chirurgie. Avantageusement, on utilise des fibres insolubles dans l'eau.

Les fibres utilisables dans la composition selon l'invention sont préférentiellement des fibres de polyamide, de poly-(p-phénylène-téréphtalamide) ou de cellulose. Leur longueur peut aller de 0.1 à 50 mm, de préférence de 0.25 à 1.6 mm et leur section peut être comprise dans un cercle de diamètre moyen pouvant aller de 5 pm à 1 mm. En particulier, on peut utiliser les fibres de polyamide commercialisées par les Etablissements P. Bonte sous le nom Polyamide 0.9 Dtex 0.3 mm, ayant un diamètre moyen de 6µm, un poids d'environ (0.9 dtex) et une longueur allant de 0,3 mm à 1,5 mm. On peut aussi utiliser les fibres de poly-(p-phénylène-téréphtalamide) de diamètre moyen de 12 µm et de longueur d'environ 1,5 mm comme celles vendues sous le nom de Kevlar Floc par la société Du Pont Fibres ou bien encore les fibres de celluloses (ou de rayonne) ayant un diamètre moyen de 50 µm et une longueur allant de 0,5 mm à 6 mm comme celles vendues sous le nom de Natural rayon flock fiber RC1BE - N003 - M04 par la société Claremont Flock. On peut également utiliser des fibres de polyéthylène comme celles vendues sous le nom de Shurt Stuff 13 099 F par la société Mini Fibers.

La concentration en fibres est fonction de l'application spécifique et du type de produit envisagé. En pratique, les fibres peuvent être présentes en une teneur allant de 0,1 % à 99 % en poids, par rapport au poids total de la composition, et de préférence de 0,5 % à 50 % en poids, et mieux de 1 % à 30 % en poids.

La composition selon l'invention contient un polymère filmogène se présentant sous la forme de particules en dispersion aqueuse, connu généralement sous le nom de latex ou pseudolatex, pour permettre une bonne cohésion et un bon maintien des fibres.

Dans la présente demande, on entend par "polymère filmogène", un polymère apte à former à lui seul ou en présence d'un agent auxiliaire de filmification, un film isolable.

Par polymère sous forme de particules en dispersion aqueuse, on entend une phase contenant de l'eau et éventuellement un composé soluble dans l'eau, dans laquelle est dispersé directement le polymère sous forme de particules.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats). Les polymères filmogènes de type radicalaires peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

On utilise de préférence des polymères filmogènes radicalaires anioniques, c'est-à-dire des polymères ayant au moins un monomère à groupement acide.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₂₀, de préférence en C₁-C₈, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle.

Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle. Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide et le N-t-octyl acrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle. Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Comme polymère filmogène acrylique utilisable selon l'invention, on peut citer ceux vendus sous les dénominations NEOCRYL XK-90®, NEOCRYL A-1070®, NEOCRYL A-1090®, NEOCRYL BT-62®, NEOCRYL A-1079®, NEOCRYL A-523® par la société ZENECA, DOW LATEX 432® par la société DOW CHEMICAL.

On peut ainsi citer, parmi les polycondensats utilisables comme polymère filmogène, les polyuréthannes anioniques, cationiques, non-ioniques ou amphotères, les polyuréthannes-acryliques, les poly-uréthannes-polyvinylpirrolidones, les polyester-polyuréthannes, les polyéther-polyuréthannes, les polyurées, les polyurée/polyuréthannes, et leurs mélanges.

Le polyuréthanne filmogène peut être, par exemple, un copolymère polyuréthanne, polyurée/uréthanne, ou polyurée, aliphatique, cycloaliphatique ou aromatique, comportant seule ou en mélange :
- au moins une séquence d'origine polyester aliphatique et/ou cycloaliphatique et/ou aromatique, et/ou,
- au moins une séquence siliconée, ramifiée ou non, par exemple polydiméthylsiloxane ou polyméthylphénylsiloxane, et/ou
- au moins une séquence comportant des groupes fluorés.

Les polyuréthannes filmogènes tels que définis dans l'invention peuvent être également obtenus à partir de polyesters, ramifiés ou non, ou d'alkydes comportant des hydrogènes mobiles que l'on modifie par réaction avec un diisocyanate et un composé organique bifonctionnel (par exemple dihydro, diamino ou hydroxyamino), comportant en plus soit un groupement acide carboxylique ou carboxylate, soit un groupement acide sulfonique ou sulfonate, soit encore un groupement amine tertiaire neutralisable ou un groupement ammonium quaternaire. Comme polyuréthanne filmogène utilisable selon l'invention on peut utiliser ceux commercialisés sous les dénominations NEOREZ R-981®, NEOREZ R-974® par la société ZENECA, les AVALURE UR-405®, AVALURE UR-410®, SANCURE 875®, SANCURE 2060®, AVALURE UR-425®, AVALURE UR-430®, SANCURE 861®, SANCURE 878®, AVALURE UR-450®par la société GOODRICH.

Parmi les polycondensats filmogènes, on peut également citer les polyesters, les polyesters amides, les polyesters à chaîne grasse, les polyamides, et les résines époxyesters.

Les polyesters peuvent être obtenus, de façon connue, par polycondensation d'acides dicarboxyliques avec des polyols, notamment des diols. L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphta-lènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K+, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique. On préfère utiliser dans les compositions objet de l'invention des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

On peut encore citer les polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthannes, les polyurées, les polyesters, les polyesteramides et/ou les alkydes. Ces polymères sont généralement appelés polymères hybrides.

Avantageusement, on peut utiliser un polymère filmogène apte à former un film vérifiant, dans les conditions de mesure définies avant les exemples, au moins une des conditions physico-chimiques suivantes :
. un module de Young inférieur à environ 200 MPa, de préférence inférieur à environ 100 MPa, et préférentiellement inférieur à 80 MPa, et/ou
. une élongation supérieure à environ 200%, et, de manière préférentielle, supérieure à 300%, et/ou
. une dureté inférieure à 110 secondes, de préférence inférieure à 70 secondes, plus préférentiellement inférieure à 55 secondes.

La dispersion comprenant un ou plusieurs polymères filmogènes peut être préparée par l'homme du métier sur base de ses connaissances générales.

La taille des particules de polymères en dispersion aqueuse peut aller de 10 à 500 nm, et est de préférence de 20 à 300 nm.

Le polymère en dispersion aqueuse peut être présent dans la composition selon l'invention en une teneur allant de 1 % à 50 % en poids, de préférence de 5 % à 40 % en poids, de matière sèche de polymères filmogènes par rapport au poids total de la composition.

La composition selon l'invention peut comprendre un agent auxiliaire de filmification favorisant la formation d'un film avec les particules du polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

Le milieu de la composition de l'invention est avantageusement un milieu aqueux contenant de l'eau. La teneur en eau dans la composition peut aller de 1 % à 70 % en poids, par rapport au poids total de la composition, et mieux de 1 % à 50 % en poids.

La composition peut contenir un agent épaississant pour faciliter l'application de la composition sur la peau.

Parmi les agents épaississants utilisables selon l'invention, on peut citer :
- les épaississants cellulosiques hydrosoluble tels que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 44001 H" par la Société Amercol,
- la gomme de guar, notamment celles vendues sous la dénomination VIDOGUM GH 175 par la société UNIPECTINE et sous la dénomination JAGUAR C par la société MEYHALL,
- la gomme de guar quaternisée vendue sous la dénomination de "Jaguar C-13-S" par la Société Meyhall,
- les gommes de guar non-ioniques comprenant des groupements hydroxyalkyle en C₁-C₆. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.
- les gommes de xanthane, de caroube, de scléroglucane, de gellane, de rhamsan, de karoya,
- les alginates, la maltodextrine, l'amidon et ses dérivés, l'acide hyaluronique et ses sels,
- les argiles, et notamment les montmorillonites, les hectorites, les laponites
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

Selon l'invention, l'agent épaississant est de préférence choisi parmi les polyuréthanes associatifs.

Les polyuréthannes associatifs sont des copolymères séquencés non ioniques comportant dans la chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

En particulier, ces polymères comportent au moins deux chaînes lipophiles hydrocarbonées, ayant de 6 à 30 atomes de carbone, séparées par une séquence hydrophile, les chaînes hydrocarbonées peuvent être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.

Les polymères peuvent être séquencés sous forme de tribloc ou multibloc. Les séquences hydrophobes peuvent donc être à chaque extrémité de la chaîne (par exemple : copolymère tribloc à séquence centrale hydrophile) ou réparties à la fois aux extrémités et dans la chaîne (copolymère multiséquencé par exemple). Les polymères peuvent être également en greffons ou en étoile.

De préférence, les polymères sont des copolymères triblocs dont la séquence hydrophile est une chaîne polyoxyéthylénée comportant de 50 à 1 000 groupements oxyéthylénés. En général les polyuréthannes associatifs comportent une liaison uréthanne entre les séquences hydrophiles, d'où l'origine du nom.

Par extension figurent aussi parmi les polyuréthannes associatifs, des polymères dont les séquence hydrophiles sont liées par d'autres liaisons chimiques, autre que la liaison polyuréthane, aux séquences lipophiles.

A titre d'exemple, des polymères associatifs utilisables dans l'invention, on peut citer le polymère C₁₆-OE₁₂₀-C₁₆ vendu par la société HULS (sous le nom Sérad FX1100, molécule à fonction uréthanne et poids moléculaire moyen en poids de 1300), OE étant un motif oxyéthyléné. Comme polymère associatif, on peut aussi utiliser aussi le Rhéolate 205 à fonction urée vendu par la société RHEOX ou encore le Rhéolate 208 ou 204. Ces polyuréthannes associatifs sont vendus sous forme pure.

Le produit DW 1206B de chez RHOM & HAAS à chaîne alkyle en C₂₀ et à liaison uréthanne, vendu à 20 % en matière sèche dans l'eau, peut aussi être utilisé.

On peut aussi utiliser des solutions ou dispersions de ces polymères notamment dans l'eau ou en milieu hydroalcoolique. A titre d'exemple, de tels polymères on peut citer, le Sérad FX1010, le Sérad FX1035 et le Serad 1070 vendus par la société HULS, le Rhéolate 255, le Rhéolate 278 et le Rhéolate 244 vendus par la société RHEOX. On peut aussi utiliser le produit DW 1206F et le DW 1206J, ainsi que l'Acrysol RM 184 ou l'Acrysol 44 de la société RHOM & HAAS, ou bien encore le Borchigel LW 44 de la société BORCHERS.

Les polymères associatifs utilisables dans l'invention sont en particulier ceux décrits dans l'article de G. Fonnum, J. Bakke et Fk. Hansen - Colloid Polym. Sci 271, 380.389 (1993).

Dans la composition selon l'invention, l'agent épaississant peut être présent en une teneur allant de 0,1 % à 20 % en poids, par rapport au poids total de la composition, et de préférence de 1 % à 10 % en poids.

Pour favoriser le séchage rapide de la composition après son application sur la peau et/ou les phanères, la composition peut comprendre des accélérateurs de séchage comme les solvants volatils et de préférence des solvants organiques volatils miscibles à l'eau, comme l'éthanol. La quantité de tels solvants organiques est choisie de telle sorte que la viscosité de la composition soit bien maintenue dans la gamme définie précédemment. Ces solvants organiques peuvent être présent dans la composition en une teneur allant jusqu'à 15 % en poids, par rapport au poids total de la composition, (notamment de 0,1 % à 15 %).et de préférence jusqu'à 10 % en poids (notamment de 0,5 % à 10 %). Par volatil, on entend un composé apte à s'évaporer au contact de la peau, à température ambiante.

La composition peut en outre comprendre d'autres ingrédients habituellement utilisés en cosmétiques. De tels ingrédients peuvent être notamment des agents plastifiants, des agents de coalescence, des charges, des matières colorantes comme les pigments ou les colorants, des cires, des tensio-actifs, des conservateurs, des huiles, des agents hydratants, des parfums qui sont bien connus de l'état de la technique. Bien entendu, l'homme du métier veillera à choisir ce ou ces éventuels additifs et/ou leur quantité, de manière telle que les propriétés avantageuses dépôt tridimensionnel sur la peau et/ou les phanères soient conservées.

La composition peut être appliquée sur la peau à l'aide de tout applicateur connu de l'homme du métier tels que les brosses, les applicateurs à embout mousse, à plume floquée, en feutre, les pinceaux, les spatules.

La composition selon l'invention peut être également appliquée sur la peau à l'aide d'un pochoir : la composition ainsi déposée dans les parties évidées du pochoir conserve sa forme après le retrait du pochoir, donnant ainsi un maquillage sur la peau très décoratif.

L'invention est illustrée plus en détail dans les exemples suivants.

### A/ Mesure de l'élongation

L'élongation du film obtenu est mesurée selon la norme ASTM Standards, volume 06.01 D 2370-92 'Standard Test Method for Tensile Properties of Organic Coatings'.

### B/ Mesure de la dureté

La dureté du film est mesurée selon la norme ASTM D-43-66, ou la norme NF-T 30-016 (octobre 1981), à l'aide d'un pendule de Persoz. Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### C/ Mesure du module de Young (ou module d'élasticité)

Le module de Young (module d'élasticité) est mesuré selon la norme ASTM Standards, volume 06.01 D 2370-92 'Standard Test Method for Tensile Properties of Organic Coatings'.

Le film déposé sur le support doit avoir une épaisseur d'environ 300 microns avant séchage. Après séchage pendant 7 jours à 21 °C et sous une humidité relative de 50%, on obtient un film ayant une épaisseur d'environ 100 microns. Les échantillons mesurés ont une largeur de 5 mm et une épaisseur de 100 microns. La distance entre les mors est de 25 mm. La vitesse de traction est de 1000 mm par minute.

### Exemple 1 :

On a préparé, par simple mélange à température ambiante, une composition de maquillage de la peau comprenant les ingrédients suivants :
- dispersion aqueuse de polyester-polyuréthane
   (49 % de matières sèches)
   (AVALURE UR-425 de la société GOODRICH) 12 g MA
- fibres de polyéthylène (Short stuff 13038F
   de la société Mini Fibers) 7 g
- Colorant qs
- Conservateurs qs
- Eau qsp 100g

Cette composition adhère bien sur la peau et confère un maquillage ayant l'aspect d'un tissu. Les fibres sont bien maintenues dans le film de polymère et le maquillage est bien résistant à l'eau et aux frottements.

## Revendications

1. Procédé cosmétique de maquillage ou de soin de la peau comprenant l'application sur la peau d'une composition cosmétique contenant, dans un milieu physiologiquement acceptable, des fibres, **caractérisé par le fait que** la composition comprend un polymère filmogène sous forme de particules en dispersion aqueuse.

2. Procédé selon la revendication 1, carcactérisé par le fait que les fibres sont choisies parmi les fibres de soie, de coton, de laine, de lin, des fibres de cellulose, de polyamide, de viscose, d'acétate notamment d'acétate de rayonne, de poly-(p-phénylène-téréphtalamide), de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères.

3. Procédé selon l'une quelconque des revendications précédentes, **caractérisé par le fait que** les fibres sont des fibres d'origine synthétique.

4. Procédé selon l'une quelconque des revendications précédentes, **caractérisé par le fait que** les fibres sont des fibres de polyamide, de poly-(p-phénylène-téréphtalamide), de cellulose ou de polyéthylène.

5. Procédé selon l'une quelconque des revendications précédentes, **caractérisé par le fait que** les fibres ont une longueur allant de 1 nm à 50 mm, de préférence de 10 nm à 5 mm.

6. Procédé selon l'une quelconque des revendications précédentes, **caractérisé par le fait que** les fibres ont une section comprise dans un cercle de diamètre moyen allant de 5 µm à 1 mm.

7. Procédé selon l'une quelconque des revendications précédentes, **caractérisé par le fait que** les fibres ont une section circulaire ou polygonale.

8. Procédé selon l'une quelconque des revendications précédentes, **caractérisé par le fait que** les fibres sont présentes en une teneur allant de 0,1 % à 99 % en poids, par rapport au poids total de la composition, de préférence de 0,5 % à 50 % en poids, et mieux de 1 % à 30 % en poids.

9. Procédé selon l'une quelconque des revendications précédentes, **caractérisé par le fait que** le polymère filmogène est choisi dans le groupe formé par les polymères radicalaires, les polycondensats, les polymères d'origine naturelle et leurs mélanges.

10. Procédé selon la revendication 9, **caractérisé par le fait que** les polymères radicalaires sont choisis dans le groupe formé par les polymères vinyliques résultant de la polymérisation de monomères choisis parmi les acides carboxyliques insaturés α,β-éthyléniques, les esters de cesdits acides, les amides de cesdits acides, les esters vinyliques, les monomères styréniques.

11. Procédé selon la revendication 9, **caractérisé par le fait que** les polycondensats sont choisis dans le groupe formé par les polyuréthanes, les polyesters, les polesters amides, les polyamides, les résines époxyesters.

12. Procédé selon la revendication 9, **caractérisé par le fait que** les polymères d'origine naturelle sont choisis dans le groupe formé par la résine shellac, la gomme de sandaraque, les dammars, les élémis, les copals, les polymères cellulosiques insolubles dans l'eau, et leurs mélanges.

13. Procédé selon l'une quelconque des revendications pécédentes, **caractérisé par le fait que** le polymère filmogène est apte à former un film vérifiant au moins une des conditions physico-chimiques suivantes :
. un module de Young inférieur à environ 200 MPa et/ou
. une élongation supérieure à environ 200%, et/ou
. une dureté inférieure à 110 secondes.

14. Procédé selon l'une quelconque des revendications précédentes, **caractérisé par le fait que** le polymère filmogène est présent en une teneur en matière sèche allant de 1 % à 50 % en poids, par rapport au poids total de la composition, et mieux de 5 % à 40 % en poids.

15. Procédé selon l'une quelconque des revendications précédentes, **caractérisé par le fait que** la composition comprend, en outre, au moins un additif choisi dans le gropue formé par les épaississants, les agents plastifiants, les agents de coalescence, les charges, les matières colorantes, les cires, les tensio-actifs, les conservateurs, les huiles, les agents hydratants, les parfums, et leurs mélanges.

16. Procédé selon l'une quelconque des revendications précédentes, **caractérisé par le fait que** la composition est appliquée sur la peau à l'aide d'un pochoir.

17. Composition cosmétique comprenant, dans un milieu physiologiquement acceptable, un polymère filmogène sous forme de particules en dispersion aqueuse et des fibres, **caractérisée par le fait que** le polymère filmogène est un polyuréthane.

18. Composition selon la revendication 17, carcactérisée par le fait que les fibres sont choisies parmi les fibres de soie, de coton, de laine, de lin, des fibres de cellulose, de polyamide, de viscose, d'acétate notamment d'acétate de rayonne, de poly-(p-phénylène-téréphtalamide), de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères.

19. Composition selon l'une des revendications 17 ou 18, **caractérisée par le fait que** les fibres sont des fibres d'origine synthétique.

20. Composition selon l'une quelconque des revendications 17 à 19, **caractérisée par le fait que** les fibres sont des fibres de polyamide, de poly-(p-phénylène-téréphtalamide), de cellulose ou de polyéthylène.

21. Composition selon l'une quelconque des revendications 17 à 20, **caractérisée par le fait que** les fibres ont une longueur allant de 1 nm à 50 mm, de préférence de 10 nm à 5 mm.

22. Composition selon l'une quelconque des revendications 17 à 21, **caractérisée par le fait que** les fibres ont une section comprise dans un cercle de diamètre moyen allant de 5 µm à 1 mm.

23. Composition selon l'une quelconque des revendications 17 à 22, **caractérisée par le fait que** les fibres ont une section circulaire ou polygonale.

24. Composition selon l'une quelconque des revendications 17 à 23, **caractérisée par le fait que** les fibres sont présentes en une teneur allant de 0,1 % à 99 % en poids, par rapport au poids total de la composition, de préférence de 0,5 % à 50 % en poids, et mieux de 1 % à 30 % en poids.

25. Composition selon l'une quelconque des revendications 17 à 24, **caractérisée par le fait que** le polyuréthane est choisi parmi les polyester-polyuréthanes et les polyéther-polyuréthanes.

26. Composition selon l'une quelconque des revendications 17 à 25, **caractérisée par le fait que** polyuréthane est anionique.

27. Composition selon l'une quelconque des revendications 17 à 26, **caractérisé par le fait que** le polymère filmogène de polyuréthane est apte à former un film vérifiant au moins une des conditions physico-chimiques suivantes :
. un module de Young inférieur à environ 200 MPa et/ou
. une élongation supérieure à environ 200%, et/ou
. une dureté inférieure à 110 secondes.

28. Composition selon l'une quelconque des revendications 17 à 27, **caractérisée par le fait que** le polyuréthane est présent en une teneur en matière sèche allant de 1 % à 50 % en poids, par rapport au poids total de la composition, et mieux de 5 % à 40 % en poids.

29. Composition selon l'une quelconque des revendications 17 à 28, **caractérisée par le fait qu'**elle comprend, en outre, au moins un additif choisi dans le groupe formé par les épaississants, les agents plastifiants, les agents de coalescence, les charges, les matières colorantes, les cires, les tensio-actifs, les conservateurs, les huiles, les agents hydratants, les parfums, et leurs mélanges.

30. Utilisation de fibres et de polymère filmogène sous forme de particules en dispersion aqueuse dans une composition cosmétique de maquillage de la peau pour obtenir un maquillage de bonne tenue et/ou résistant à l'eau et/ou aux frottements et/ou camouflant la peau.

## Claims

1. Cosmetic make-up or care process for the skin, comprising the application to the skin of a cosmetic composition containing fibres in a physiologically acceptable medium, **characterized in that** the composition comprises a film-forming polymer in the form of particles in aqueous dispersion.

2. Process according to Claim 1, **characterized in that** the fibres are chosen from silk fibre, cotton fibre, wool fibre, flax fibre, cellulose fibres, polyamide fibre, viscose fibre, acetate fibre in particular rayon acetate fibre, poly-p-phenylene terephthalamide fibre, acrylic polymer fibre in particular polymethyl methacrylate fibre or poly(2-hydroxyethyl methacrylate) fibre, polyolefin fibre, and in particular polyethylene or polypropylene fibre, glass fibre, silica fibre, carbon fibre in particular in graphite form, polytetrafluoroethylene fibre, insoluble collagen fibre, polyester fibre, polyvinyl chloride or polyvinylidene chloride fibre, polyvinyl alcohol fibre, polyacrylonitrile fibre, chitosan fibre, polyurethane fibre; polyethylene phthalate fibre, or fibres formed from a mixture of polymers.

3. Process according to either of the preceding claims, **characterized in that** the fibres are fibres of synthetic origin.

4. Process according to any one of the preceding claims, **characterized in that** the fibres are polyamide fibres, poly(p-phenylene terephthalamide) fibres, cellulose fibres or polyethylene fibres.

5. Process according to any one of the preceding claims, **characterized in that** the fibres have a length ranging from 1 nm to 50 mm, preferably from 10 nm to 5 mm.

6. Process according to any one of the preceding claims, **characterized in that** the fibres have a cross section included in a circle with an average diameter ranging from 5 µm to 1 mm.

7. Process according to any one of the preceding claims, **characterized in that** the fibres have a circular or polygonal cross section.

8. Process according to any one of the preceding claims, **characterized in that** the fibres are present in a content ranging from 0.1% to 99% by weight, relative to the total weight of the composition, preferably from 0.5% to 50% by weight and better still from 1% to 30% by weight.

9. Process according to any one of the preceding claims, **characterized in that** the film-forming polymer is chosen from the group formed by radical-mediated polymers, polycondensates and polymers of natural origin, and mixtures thereof.

10. Process according to Claim 9, **characterized in that** the radical-mediated polymers are chosen from the group formed by vinyl polymers resulting from the polymerization of monomers chosen from α,β-ethylenic unsaturated carboxylic acids, the esters of these said acids, the amides of these said acids, vinyl esters and styrene monomers.

11. Process according to Claim 9, **characterized in that** the polycondensates are chosen from the group formed by polyurethanes, polyesters, polyesteramides, polyamides and epoxyester resins.

12. Process according to Claim 9, **characterized in that** the polymers of natural origin are chosen from the group formed by shellac resin, sandarac gum, dammar resins, elemis gums, copal resins and water-insoluble cellulose polymers, and mixtures thereof.

13. Process according to any one of the preceding claims, **characterized in that** the film-forming polymer is capable of forming a film which satisfies at least one of the following physicochemical conditions:
. a Young's modulus of less than about 200 MPa and/or
. an elongation of greater than about 200%, and/or
. a hardness of less than 110 seconds.

14. Process according to any one of the preceding claims, **characterized in that** the film-forming polymer is present in a solids content ranging from 1% to 50% by weight, relative to the total weight of the composition, and better still from 5% to 40% by weight.

15. Process according to any one of the preceding claims, **characterized in that** the composition also comprises at least one additive chosen from the group formed by thickeners, plasticizers, coalescers, fillers, dyestuffs, waxes, surfactants, preserving agents, oils, moisturizers and fragrances, and mixtures thereof.

16. Process according to any one of the preceding claims, **characterized in that** the composition is applied to the skin with the aid of a stencil.

17. Cosmetic composition comprising, in a physiologically acceptable medium, a film-forming polymer in the form of particles in aqueous dispersion and fibres, **characterized in that** the film-forming polymer is a polyurethane.

18. Composition according to Claim 17, **characterized in that** the fibres are chosen from silk fibre, cotton fibre, wool fibre, flax fibre, cellulose fibres, polyamide fibre, viscose fibre, acetate fibre in particular rayon acetate fibre, poly-p-phenylene terephthalamide fibre, acrylic polymer fibre in particular polymethyl methacrylate fibre or poly(2-hydroxyethyl methacrylate) fibre, polyolefin fibre, and in particular polyethylene or polypropylene fibre, glass fibre, silica fibre, carbon fibre in particular in graphite form, polytetrafluoroethylene fibre, insoluble collagen fibre, polyester fibre, polyvinyl chloride or polyvinylidene chloride fibre, polyvinyl alcohol fibre, polyacrylonitrile fibre, chitosan fibre, polyurethane fibre, polyethylene phthalate fibre, or fibres formed from a mixture of polymers.

19. Composition according to either of Claims 17 and 18, **characterized in that** the fibres are fibres of synthetic origin.

20. Composition according to any one of Claims 17 to 19, **characterized in that** the fibres are polyamide fibres, poly(p-phenylene terephthalamide) fibres, cellulose fibres or polyethylene fibres.

21. Composition according to any one of Claims 17 to 20, **characterized in that** the fibres have a length ranging from 1 nm to 50 mm, preferably from 10 nm to 5 mm.

22. Composition according to any one of Claims 17 to 21, **characterized in that** the fibres have a cross section included in a circle with an average diameter ranging from 5 µm to 1 mm.

23. Composition according to any one of Claims 17 to 22, **characterized in that** the fibres have a circular or polygonal cross section.

24. Composition according to any one of Claims 17 to 23, **characterized in that** the fibres are present in a content ranging from 0.1% to 99% by weight, relative to the total weight of the composition, preferably from 0.5% to 50% by weight and better still from 1% to 30% by weight.

25. Composition according to any one of Claims 17 to 24, **characterized in that** the polyurethane is chosen from polyester-polyurethanes and polyether-polyurethanes.

26. Composition according to any one of Claims 17 to 25, **characterized in that** the polyurethane is anionic.

27. Composition according to any one of Claims 17 to 26, **characterized in that** the film-forming polyurethane polymer is capable of forming a film which satisfies at least one of the following physicochemical conditions:
. a Young's modulus of less than about 200 MPa and/or
. an elongation of greater than about 200%, and/or
. a hardness of less than 110 seconds.

28. Composition according to any one of Claims 17 to 27, **characterized in that** the polyurethane is present in a solids content ranging from 1% to 50% by weight, relative to the total weight of the composition, and better still from 5% to 40% by weight.

29. Composition according to any one of Claims 17 to 28, **characterized in that** the composition also comprises at least one additive chosen from the group formed by thickeners, plasticizers, coalescers, fillers, dyestuffs, waxes, surfactants, preserving agents, oils, moisturizers and fragrances, and mixtures thereof.

30. Use of fibres and of film-forming polymer in the form of particles in aqueous dispersion in a cosmetic make-up composition for the skin, in order to obtain a make-up which has good staying power and/or which is resistant to water and/or to rubbing and/or which camouflages the skin.

## Patentansprüche

1. Kosmetisches Verfahren zum Schminken oder zur Pflege der Haut, das umfasst, auf die Haut eine kosmetische Zusammensetzung aufzubringen, die in einem physiologisch akzeptablen Medium Fasern enthält, **dadurch gekennzeichnet, dass** die Zusammensetzung ein filmbildendes Polymer in Form von Partikeln in wässriger Dispersion enthält.

2. Verfahren nach Anspruch 1, **dadurch gekennzeichnet, dass** die Fasern unter den Seidefasern, Baumwollfasern, Leinenfasern, Cellulosefasern, Polyamidfasern, Viskosefasern, Acetatfasern, insbesondere aus Reyonacetat, Poly-p-phenylenterephthalamidfasern, Acrylfasern, insbesondere aus Polymethylmethacrylat oder Poly-2-hydroxyethylmethacrylat, Polyolefinfasern und insbesondere aus Polyethylen oder Polypropylen, Glasfasern, Siliciurndioxidfasern, Kohlenstofffasern, insbesondere Kohlenstoff in Form von Graphit, Polytetrafluorethylenfasern, Fasern aus unlöslichem Kollagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern und Fasern aus Polymergemischen ausgewählt sind.

3. Verfahren nach einem der vorhergehenden Ansprüche, **dadurch gekennzeichnet, dass** es sich bei den Fasern um Fasern synthetischer Herkunft handelt.

4. Verfahren nach einem der vorhergehenden Ansprüche, **dadurch gekennzeichnet, dass** die Fasern Polyamidfasern, Poly-p-phenylenterephthalamidfasern, Cellulosefasern oder Polyethylenfasern sind.

5. Verfahren nach einem der vorhergehenden Ansprüche, **dadurch gekennzeichnet, dass** die Fasern eine Länge von 1 nm bis 50 mm und vorzugsweise 10 nm bis 5 mm besitzen.

6. Verfahren nach einem der vorhergehenden Ansprüche, **dadurch gekennzeichnet, dass** die Fasern einen Querschnitt besitzen, der in einen Kreis mit einem mittleren Durchmesser von 5 µm bis 1. mm einbeschrieben werden kann.

7. Verfahren nach einem der vorhergehenden Ansprüche, **dadurch gekennzeichnet, dass** die Fasern einen kreisförmigen oder polygonalen Querschnitt besitzen.

8. Verfahren nach einem der vorhergehenden Ansprüche, **dadurch gekennzeichnet, dass** die Fasern in einer Menge von 0,1 bis 99 Gew.-%, bezogen auf das Gesamtgewicht der Zusammensetzung, vorzugsweise 0,5 bis 50 Gew.-% und besser 1 bis 30 Gew.-%, enthalten sind.

9. Verfahren nach einem der vorhergehenden Ansprüche, **dadurch gekennzeichnet, dass** das filmbildende Polymer unter den durch radikalische Polymerisation hergestellten Polymeren, Polykondensaten, Polymeren natürlicher Herkunft und deren Gemischen ausgewählt sind.

10. Verfahren nach Anspruch 9, **dadurch gekennzeichnet, dass** die durch radikalische Polymerisation hergestellten Polymere unter den Vinyl polymeren ausgewählt sind, die bei der Polymerisation von Monomeren gebildet werden, die unter den α,β-ethylenisch ungesättigten Carbonsäuren, den Estern dieser Säuren, den Amiden dieser Säuren, Vinylestern und Styrolmonomeren ausgewählt sind.

11. Verfahren nach Anspruch 9, **dadurch gekennzeichnet, dass** die Polykondensate unter den Polyurethanen, Polyestern, Polyesteramiden, Polyamiden und Epoxyesterharzen ausgewählt sind.

12. Verfahren nach Anspruch 9, **dadurch gekennzeichnet, dass** die Polymere natürlicher Herkunft unter Schellack, Sandarak, Dammarharzen, Elemi, Kopalen, Cellulosepolymeren, die in Wasser unlöslich sind, und deren Gemischen ausgewählt sind.

13. Verfahren nach einem der vorhergehenden Ansprüche, **dadurch gekennzeichnet, dass** das filmbildende Polymer befähigt ist, einen Film zu bilden, der mindestens eine der folgenden physikalisch-chemischen Eigenschaften erfüllt:
- Young-Modul unter etwa 200 MPa und/oder
- Dehnung über etwa 200 % und/oder
- Härte unter 110 Sekunden

14. Verfahren nach einem der vorhergehenden Ansprüche, **dadurch gekennzeichnet, dass** das filmbildende Polymer in einem Trockensubstanzgehalt von 1 bis 50 Gew.-%, bezogen auf das Gesamtgewicht der Zusammensetzung, und besser 5 bis 40 Gew.-% vorliegt.

15. Verfahren nach einem der vorhergehenden Ansprüche, **dadurch gekennzeichnet, dass** die Zusammensetzung außerdem mindestens einen Zusatzstoff enthält, der unter den Verdickungsmitteln, Weichmachern, Koaleszenzmitteln, Füllstoffen, Farbmitteln, Wachsen, grenzflächenaktiven Stoffen, Konservierungsmitteln, Ölen, Hydratisierungsmitteln, Parfums und deren Gemischen ausgewählt ist.

16. Verfahren nach einem der vorhergehenden Ansprüche, **dadurch gekennzeichnet, dass** die Zusammensetzung mit Hilfe einer Schablone auf die Haut aufgebracht wird.

17. Kosmetische Zusammensetzung, die in einem physiologisch akzeptablen Medium ein filmbildendes Polymer in Form von Partikeln einer wässrigen Dispersion und Fasern enthält, **dadurch gekennzeichnet, dass** das filmbildende Polymer ein Polyurethan ist.

18. Zusammensetzung nach Anspruch 17, **dadurch gekennzeichnet, dass** die Fasern unter den Seidefasern, Baumwollfasern, Leinenfasern, Cellulosefasern, Polyamidfasern, Viskosefasern, Acetatfasern, insbesondere aus Reyonacetat, Poly-p-phenylenterephthalamidfasern, Acrylfasern, insbesondere aus Polymethylmethacrylat oder Poly-2-hydroxyethylmethacrylat, Polyolefinfasern und insbesondere aus Polyethylen oder Polypropylen, Glasfasern, Siliciumdioxidfasern, Kohlenstofffasern, insbesondere Kohlenstoff in Form von Graphit, Polytetrafluorethylenfasern, Fasern aus unlöslichem Kollagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern und Fasern aus Polymergemischen ausgewählt sind.

19. Zusammensetzung nach einem der Ansprüche 17 oder 18, **dadurch gekennzeichnet, dass** die Fasern Fasern synthetischer Herkunft sind.

20. Zusammensetzung nach einem der Ansprüche 17 bis 19, **dadurch gekennzeichnet, dass** es sich bei den Fasern um Polyamidfasern, Poly-p-phenylenterephthalamidfasern, Cellulosefasern oder Polyethylenfasern handelt.

21. Zusammensetzung nach einem der Ansprüche 17 bis 20, **dadurch gekennzeichnet, dass** die Fasern eine Länge von 1 nm bis 50 mm und vorzugsweise 10 nm bis 5 mm besitzen.

22. Zusammensetzung nach einem der Ansprüche 17 bis 21, **dadurch gekennzeichnet, dass** die Fasern einen Querschnitt aufweisen, der in einen Kreis mit einem mittleren Durchmesser von 5 µm bis 1 mm einbeschrieben werden kann.

23. Zusammensetzung nach einem der Ansprüche 17 bis 22, **dadurch gekennzeichnet, dass** die Fasern einen kreisförmigen oder polygonalen Querschnitt besitzen.

24. Zusammensetzung nach einem der Ansprüche 17 bis 23, **dadurch gekennzeichnet, dass** die Fasern in einer Menge von 0,1 bis 99 Gew.-%, bezogen auf das Gesamtgewicht der Zusammensetzung, vorzugsweise 0,5 bis 50 Gew.-% und besser 1 bis 30 Gew.-% enthalten sind.

25. Zusammensetzung nah einem der Ansprüche 10 bis 24, **dadurch gekennzeichnet, dass** das Polyurethan unter den Polyesterpolyurethanen und Polyetherpolyurethanen ausgewählt ist.

26. Zusammensetzung nach einem der Ansprüche 17 bis 25, **dadurch gekennzeichnet, dass** das Polyurethan anionisch ist.

27. Zusammensetzung nach einem der Ansprüche 17 bis 26, **dadurch gekennzeichnet, dass** das filmbildende Polyurethan befähigt ist, einen Film zu bilden, der mindestens eine der fol-genden physikalisch-chemischen Eigenschaften aufweist:
- Young-Modul unter etwa 200 MPa und/oder
- Dehnung über etwa 200 % und/ oder
- Härte unter 110 Sekunden

28. Zusammensetzung nach einem der Ansprüche 17 bis 27, **dadurch gekennzeichnet, dass** das Polyurethan in einem Trockensubstanzgehalt von 1 bis 50 Gew.-%, bezogen auf das Gesamtgewicht der Zusammensetzung, und besser 5 bis 40 Gew.-% enthalten ist.

29. Zusammensetzung nach einem der Ansprüche 17 bis 28, **dadurch gekennzeichnet, dass** sie ferner mindestens einen Zusatzstoff enthält, der unter den Verdickungsmitteln, Weichmachern, Koaleszenzmitteln, Füllstoffen, Farbmitteln, Wachsen, grenzflächenaktiven Stoffen, Konservierungsmitteln, Ölen, Hydratisierungsmitteln, Parfums und deren Gemischen ausgewählt ist.

30. Verwendung von Fasern und eines filmbildenden Polymers in Form von Partikeln in wässriger Dispersion in der kosmetischen Zusammensetzung zum Schminken der Haut, um eine Schminke mit guter Haftung und/oder eine Schminke, die wasserbeständig und/oder reibungsbeständig ist, und/oder eine Schminke, die die Haut abdeckt, zu erhalten.
